# EUROPEAN PATENT APPLICATION

(11) **EP 4 729 627 A1**
(43) Date of publication of application: **22.04.2026**
(21) Application number: 23941298.4
(22) Date of filing: 30.10.2023
(51) Int. Cl.: C12Q 1/6809, C12N 15/11, C12Q 1/6888, C12Q 1/6874, G16B 20/20, G16B 30/10, C40B 40/06

(54) **MULTIPLE SINGLE-NUCLEOTIDE POLYMORPHISM-BASED 10K LIQUID PHASE CHIP FOR PIG, AND USE THEREOF**

(30) Priority: 15.06.2023 CN 202310707878
(71) Applicant: China Agricultural University, Beijing 100193 (CN)
(72) Inventor: DING, Xiangdong, Beijing 100193 (CN); LIU, Huatao, Beijing 100193 (CN); LI, Shujuan, Beijing 100193 (CN); ZHANG, Zipeng, Beijing 100193 (CN); DOU, Hehe, Beijing 100193 (CN); SHAO, Yuru, Beijing 100193 (CN); WANG, Chuduan, Beijing 100193 (CN)
(74) Representative: Maiwald GmbH
(86) International application number: PCT/CN2023/127963
(87) International publication number: WO 2024/255074

(57) **Abstract**

This invention relates to the field of genetic molecular breeding, specifically to a pig 10K liquid-phase chip based on multiple single nucleotide-polymorphism and its application. The present invention, while adhering to the basic principles of liquid phase chip design, adds new markers to existing chips and optimizes probes using multiple single nucleotide-polymorphism technology. This results in the generation of more SNP markers with high genotyping quality and moderate linkage disequilibrium with target SNP loci within the probe region, thereby increasing the effective marker count of the chip. The mSNP liquid phase chip of the present invention increases the number of detectable SNPs to 1.5-2 times the number of target SNP markers, addressing the issue of existing chips that only contain target SNPs and cannot provide mSNP markers, without increasing the cost. By using genotype imputation technology, genotype data from the chip of the present invention is imputed into mainstream 50K chips for genomic selection, achieving molecular breeding accuracy comparable to or similar to that of the 50K chips, thereby reducing the cost of pig molecular breeding.

## Description

### Technical Field

This invention relates to the field of biological breeding, specifically to a 10K liquid-phase chip for pigs based on multiple single nucleotide polymorphisms (mSNP) and its application.

### Background Technology

With the rapid advancement of molecular biology techniques, biotechnology breeding centered on genomic selection technology has begun to be extensively researched and applied in dairy cattle, pigs, chickens, crops, forestry, and aquatic organisms, bringing about a significant revolution in animal and poultry breeding and becoming a powerful driving force for its progress. Currently, genomic selection is also a breeding technology vigorously promoted in China's seed industry revitalization plan. A prerequisite for genomic selection is the rapid, high-throughput genotyping of individuals. Single nucleotide polymorphism (SNP) is considered the best molecular marker currently due to its large quantity, widespread distribution across the genome, and relative ease for large-scale rapid screening and genotyping. Genomic selection involves calculating the genomic breeding values of individuals using SNP markers that cover the entire genome, thereby selecting superior individuals. As molecular detection technology advances, SNP chips capable of high-throughput genotyping have emerged.

Theoretical research and breeding practice show that a 50K chip containing approximately 50,000 SNP markers is effective for molecular breeding in animals, and it is currently the mainstream technology for genomic selection. However, in animal breeding, especially in pigs with large population sizes, early selection of piglets is required to determine the individuals that will enter performance testing, ultimately selecting the outstanding individuals to enter the core breeding population. However, in the early selection of piglets, only parental information can be utilized, and individuals from the same litter cannot be distinguished in terms of genetic merit due to the same parental information. Research shows that genomic selection can improve the accuracy of early piglet selection. "Dragon breeds nine sons, each different" , and genomic information can help select the best individuals from the same litter. This requires large-scale genotyping of piglets. For example, in a breeding farm with 1,000 sows, to achieve ideal genomic selection results, at least 22,000 piglets need to be tested annually. The costs for solid-phase 50K chips and liquid-phase 50K chips would be 3.96 million and 2.97 million, respectively. Although the liquid phase has significantly reduced chip testing costs, it remains a substantial burden, greatly limiting the implementation of molecular breeding in China's pig industry. Some companies test only a small number of individuals to save costs, introducing a lot of randomness and greatly reducing breeding effectiveness. If a low-density chip such as 10K (containing around 10,000 markers) is used for early detection, the cost of genotyping for early selection can be greatly reduced due to its low price. Moreover, using genotype imputation technology, the 10K chip marker genotypes can be imputed to the 50K chip, maintaining high accuracy and ensuring the same early selection accuracy as testing with the 50K chip.

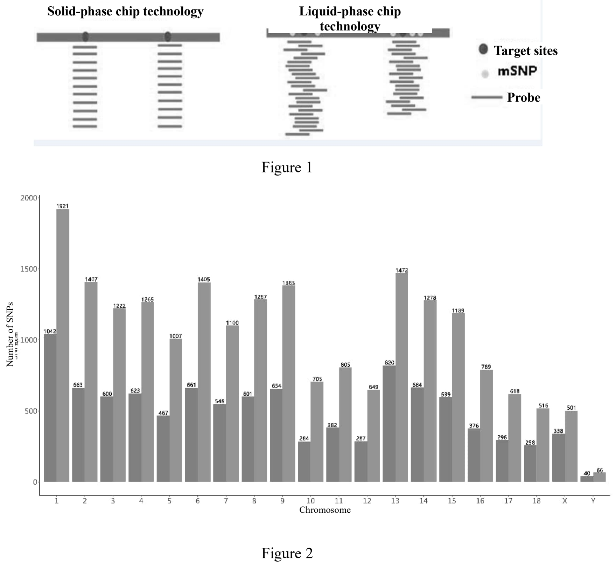

However, there is currently no commercialized 10K chip for pig breeding. The inventors have developed a 10K low-density chip based on solid-phase chip technology, which has been granted a patent (Patent No.: ZL201711190317.6, containing 8,846 markers). But due to the inflexibility of solid-phase chips, strict sample size requirements (multiples of 12 or 24), and high customization costs, large-scale use of the chip is limited. Unlike solid-phase chips, liquid-phase chips based on genotyping by target sequencing (GBTS) technology have advantages such as easy addition or removal of markers, no sample size requirements and low customization costs. Figure 1 shows that GBTS technology mainly performs multiple sequencing of the target site and its upstream and downstream regions to ensure the quality of target site genotyping. Additionally, liquid-phase chips can also genotype polymorphic sites upstream and downstream of the target site, known as multiple single nucleotide polymorphism clusters (mSNPs or multiple dispersed nucleotide polymorphisms, MNPs). For mSNPs with moderate linkage disequilibrium, markers upstream and downstream of the target site can provide additional information. Therefore, multiple single nucleotide-polymorphism technology technology can clearly increase the number of effective mSNP markers and enhance the information content of liquid-phase chips, making full use of the GBTS technology's advantages without increasing target site SNPs and testing costs-something solid-phase chip technology cannot achieve.

### Summary of the Invention

Therefore, based on the developed pig 9K solid-phase SNP chip (Patent No.: ZL201711190317.6) and considering the characteristics of the pig genome, the present invention utilizes multiple single nucleotide-polymorphism technology to optimize the design of target site probes and develops a pig 10K liquid phase chip (referred to as the 10K liquid phase chip) based on multiple single nucleotide-polymorphism. This chip is intended for pig genomic breeding and aims to reduce the cost of pig molecular breeding.

This invention has developed a 10K liquid-phase chip for pigs and also provides a method for selecting SNP markers and preparing probes for the chip. Using the chip designed by this invention can save costs on pig genomic selection and maximize the use of mSNP marker information upstream and downstream of target sites, improving the efficiency of genetic analysis and molecular breeding in pigs.

Specifically, the objectives of this invention are to provide the following:
**The first aspect provides a method for selecting SNP markers and preparing probes.** Based on whole-genome sequencing data of Duroc, Large White, and Landrace pigs (version 11.1 reference genome), 10,200 target SNPs are mined and selected. Probes are then designed and optimized based on the principles of probe design, resulting in 15,572 probes.

The method includes the following steps:

### Step 1: Target SNP marker selection

(1) Preliminary selection of target SNP markers. According to the method described in ZL201711190317.6, based on whole-genome sequencing data from Duroc, Large White, and Landrace pig breeds, aligned to the pig 11.1 reference genome, markers are selected for targeted capture sites, i.e., target SNPs, based on marker spacing and gene function annotations. The principles for selecting target SNPs are: ① Uniform distribution across chromosomes, with denser distribution at both ends of chromosomes; ② Marker spacing greater than 280Kb; ③ Important candidate loci for growth, reproduction, feed efficiency, meat quality, and body size traits; ④ SNP markers preferably located in QTL regions related to economic traits, as determined by comparison with the QTLdb database. As a result, 8,846 markers described in ZL201711190317.6 were obtained as target sites.
(2) Supplementation and optimization of target SNPs. Based on the initial screening, supplement and optimize markers with high polymorphism and moderate regional linkage disequilibrium. The principles are as follows: ① Polymorphism primarily considers MAF > 0.35 in Duroc, Landrace, and Large White pigs; ② The average linkage disequilibrium (r2) with adjacent SNP markers is below 0.85.

In regions where the marker spacing is greater than 280 kb (with an average spacing of 279 kb in the 9K solid-phase chip), select markers that meet the above SNP principles to supplement and optimize the target SNPs. This results in a total of 2,243 additional target SNPs that meet the requirements compared to the pig 9K solid-phase SNP chip.

### Step 2: Design probes based on the target site SNPs

Probes are optimized and designed using multiple single nucleotide-polymorphism. For each target SNP, 1-4 probes of 110bp length are designed, with each probe covering the target SNP. The total coverage of probes centered on the target SNP is 165bp in length. The principles of probe design are: 1) Select probes with content between 30%-80%; 2) Select regions with a homology number ≤5; 3) Exclude regions containing SSR or N regions in the probe.

**Step 3: Through multiple sequencing and hybridization of probes, select probes containing high-quality mSNPs, and optimize the probes.**

Probes are sequenced and hybridized to detect the genotyping quality of target sites and mSNPs. Set a missing rate of NA<0.1, a minimum allele frequency (MAF) ≥0.05, and heterozygosity (Het) <0.5 as standards to screen mSNPs, with removal of those that do not meet the standards. If the probe does not meet the genotype quality control requirements of mSNPs, delete the probe and the corresponding target site SNP. The mSNPs that meet the quality inspection requirements are finally used as 10K mSNP liquid-phase chip loci.

The method described in step three is designed to ensure the optimal quantity and quality of mSNP markers (including target SNPs) under the same target SNP probe.

According to the quality requirements for probes and target SNPs (missing rate NA < 0.1, minimum allele frequency MAF ≥ 0.05, heterozygosity Het < 0.5), 1,089 target SNPs from the 9K solid-phase chip did not meet the requirements and were removed. The newly added 2,443 target SNPs and mSNPs meet the requirements. This invention ultimately includes 10,200 target SNPs and 15,572 probes.

From the above, it is evident that the target site SNPs selected by the present invention are an optimization and update based on the 9K, where target site SNPs with poor probe quality and a low number of mSNP markers upstream and downstream in the pig 9K solid-phase SNP chip have been eliminated, and new target sites have been added in intervals greater than 280KB within the pig 9K solid-phase SNP chip.

**In the second aspect,** based on the 15,572 probes designed in the first aspect, this invention synthesizes the pig 10K probes, mixes them in equimolar amounts, and dissolves them in a buffer solution of 3 pmol/mL to obtain the pig 10K probe mixture, for subsequent probe sequencing and hybridization. It is known as the mSNP liquid-phase chip.

Step 1: Probe preparation. The synthesized pig 10K probes are mixed in equimolar amounts and dissolved in a buffer solution of 3 pmol/mL to obtain the pig 10K probe mixture.

Step 2: Preparation of probe hybridization solution. According to the library type, this invention uses Pooled, barcoded library, GenoBaits Block I, GenoBaits Block II for ILM/MGI to prepare the pig 10K probe hybridization solution, used for subsequent DNA library hybridization capture.

**In the third aspect,** the liquid-phase chip developed in the second aspect is used to detect the genotype of individual pigs.

The method includes the following steps:
Step 1: Obtaining and extracting genomic DNA from the pig sample;
Step 2: Constructing a pig cDNA library;
Step 3: Hybridizing and sequencing the library with the probes developed in this invention;
Step 4: Genotyping the mSNPs based on the sequencing data to determine the genotype of all liquid-phase chip marker loci for the individual.

Compared with the prior art, the development method of the 10K liquid-phase chip for pigs provided by this invention has the following beneficial effects:
The present invention provides a 10K liquid-phase chip probe for pigs based on targeted capture sequencing for genotyping. The probe design considers the distribution of captured SNP loci across the genome, locus polymorphism, mSNP marker quality, and other issues. In the Duroc, Landrace, and Large White pig populations, the target site MAF requirement is greater than 0.35, effectively avoiding the problems of uneven marker density and poor polymorphism that may arise with reduced-representation genome sequencing genotyping technology.

This invention considers the issue of linkage disequilibrium among mSNP markers within the same target site probe (1-4 probes), generating more high-quality mSNPs with moderate linkage disequilibrium within the probe region, increasing the overall polymorphism of SNP markers within the probe region and expanding the number of detectable SNPs to 1.5-2 times the number of loci. This solves the problem of the relatively small number of high-quality and effective mSNP markers in traditional liquid-phase chips without increasing costs.

This invention helps reduce the cost of pig genomic selection while maintaining the accuracy of genomic selection. Compared with the current mainstream 50K chip, the testing cost of this invention is 1/3 of that of the 50K solid-phase chip and 1/2 of the 50K liquid-phase chip. This invention considers compatibility with 50K solid-phase chips and 50K liquid-phase chips, allowing the 10K liquid-phase chip genotype data to be filled into the 50K chip through genotype imputation technology with high accuracy. The imputed genotype data used for genomic selection can achieve the same accuracy as the 50K chip, greatly reducing the cost of molecular breeding and enabling large-scale use in genomic breeding.

### Description of the Drawings

Figure 1 is a schematic diagram of solid-phase chip and liquid-phase chip technologies.
Figure 2 shows the distribution of target sites (dark gray) and mSNPs (light gray) of the 10K mSNP liquid-phase chip across various chromosomes in pigs.
Figure 3 shows the chromosomal density distribution of target sites (A) and all mSNPs containing the target sites (B) on the 10K mSNP liquid-phase chip.
Figure 4 shows the number of SNP and mSNP markers of the 10K liquid-phase chip after genotype quality control.
Figure 5 shows the distribution of target sites (A) and all mSNPs (including target sites) (B) across chromosomes after quality control.
Figure 6 shows the filling accuracy of different chromosomes (a) and MAF intervals (b) when 10K liquid-phase chip genotypes are filled up to 50K.

### Specific Method of Embodiment

Below, the specific embodiments of the invention are described in further detail in conjunction with the accompanying drawings and examples. The following examples are intended to illustrate the invention but are not intended to limit its scope.

### Example 1: Screening Method for SNP Markers and Probe Preparation Required for the Invention

### Step 1: Target SNP marker selection

(1) Preliminary selection of target SNP markers. According to the method described in ZL201711190317.6, based on whole-genome sequencing data from Duroc, Large White, and Landrace pig breeds, aligned to the pig 11.1 reference genome, markers are selected for targeted capture sites, i.e., target SNPs, based on marker spacing and gene function annotations. The principles for selecting target SNPs are: ① Uniform distribution across chromosomes, with denser distribution at both ends of chromosomes; ② Marker spacing greater than 280Kb; ③ Important candidate loci for growth, reproduction, feed efficiency, meat quality, and body size traits; ④ SNP markers preferably located in QTL regions related to economic traits, as determined by comparison with the QTLdb database. As a result, 8,846 markers described in ZL201711190317.6 were obtained as target sites.
(2)Supplementation and optimization of target SNPs.

Based on the initial screening, supplement and optimize markers with high polymorphism and moderate regional linkage disequilibrium. The principles are as follows: ① Polymorphism primarily considers MAF > 0.35 in Duroc, Landrace, and Large White pigs; ② The average linkage disequilibrium (r2) with adjacent SNP markers is below 0.85.

In regions where the marker spacing is greater than 280 kb (with an average spacing of 279 kb in the 9K solid-phase chip), select markers that meet the above SNP principles to supplement and optimize the target SNPs. This results in a total of 2,243 additional target SNPs that meet the requirements.

### Step 2: Design probes based on the target site SNPs

Probes are optimized and designed using multiple single nucleotide-polymorphism. For each target SNP, 1-4 probes of 110bp length are designed, with each probe covering the target SNP. The total coverage of probes centered on the target SNP is 165bp in length. The principles of probe design are: 1) Select probes with content between 30%-80%; 2) Select regions with a homology number ≤5; 3) Exclude regions containing SSR or N regions in the probe.

**Step 3: Through multiple sequencing and hybridization of probes, select probes containing high-quality mSNPs, and optimize the probes.**

Probes are hybridized and sequenced to assess the genotyping quality of mSNPs within the target site SNP and its upstream and downstream probe coverage range. The criteria for selecting mSNPs include setting a missing rate of NA<0.1, a minimum allele frequency (MAF) ≥0.05, and heterozygosity (Het) <0.5 as standards to screen mSNPs, with removal of those that do not meet the standards. If the probe does not meet the genotype quality control requirements of mSNPs, delete the probe and the corresponding target site SNP. The SNPs that meet the quality inspection requirements are finally used as 10K mSNP liquid-phase chip loci.

The 9K solid-phase chip technology used in pig genotyping only considers the genotyping quality of target site SNPs without considering the mSNP quality upstream and downstream of the target site SNP. A total of 1089 target sites that did not meet the probe quality and mSNP selection standards were removed. All 2443 newly supplemented target site SNPs met the probe and mSNP quality requirements. The pig 10K mSNP liquid phase chip developed by the present invention has 10,200 target loci and 15,572 probes designed. Table 1 shows the number of target site SNPs deleted and added on each chromosome in the 9K solid-phase chip. In addition, the invention adopts multiplex multiple single nucleotide-polymorphism technology, where multiple mSNPs are present within a 200 bp region around each target site SNP, generating a total of approximately 20580 high-quality mSNPs with 15572 probes. The distribution of target sites and mSNPs (including target sites) across chromosomes is shown in Figure 2.

**Table 1 shows the number of target site SNPs deleted and added on each chromosome in the 9K solid-phase chip.**

| Chromosome | Number of markers deleted from the 9K solid-phase chip | Number of newly added target sites in the 9K solid-phase chip |
|---|---|---|
| 1 | 93 | 290 |
| 2 | 74 | 157 |
| 3 | 52 | 123 |
| 4 | 37 | 123 |
| 5 | 52 | 114 |
| 6 | 187 | 226 |
| 7 | 58 | 93 |
| 8 | 64 | 128 |
| 9 | 61 | 122 |
| 10 | 27 | 76 |
| 11 | 28 | 64 |
| 12 | 23 | 73 |
| 13 | 82 | 239 |
| 14 | 70 | 144 |
| 15 | 69 | 155 |
| 16 | 22 | 67 |
| 17 | 14 | 61 |
| 18 | 19 | 56 |
| X | 48 | 117 |
| Y | 9 | 15 |
| Total | 1089 | 2443 |

### Example 2: Preparation of the 10K Liquid-Phase Chip

This example demonstrates the preparation process of the 10K liquid-phase chip according to the invention. The specific steps are as follows:

### Step 1: Probe Preparation

Based on the determination of pig 10K probes in Example 1, probes are synthesized, mixed equimolarly, and diluted with EDTA and Tris-HCl (TE buffer) solution to prepare a 3 pmol/mL pig 10K probe mixture, which is used for subsequent sequencing and hybridization.

### 1. Preparation of TE buffer

1×TE Buffer
Composition and concentration: 10 mM Tris-HCl, 1 mM EDTA, pH = 8.0
Preparation volume: 500 mL
Preparation method: Measure the following solutions into a 500 mL beaker:
   1 M Tris-HCl Buffer, pH = 8.0, 5 mL
   0.5 M EDTA, pH = 8.0, 1 mL

Add about 400ml dd H₂O to the beaker, mix well; then dilute the solution to 500ml, and sterilize at high temperature and pressure; store at room temperature.

2. Use the prepared TE buffer to dissolve the pig 10K probes and prepare a 3 pmol/mL pig 10K probe mixture for subsequent sequencing and hybridization.

### Step 2: Preparation of probe hybridization solution

1. According to the library type, mix the following reagents in a 1.5mL PCR tube, with the composition and volume as follows:

| | |
|---|---|
| Library | 0.6 µL |
| GenoBaits Block I | 5 µL |
| GenoBaits Block II for ILM/MGI | 2 µL |
| Invention probe | 300 ng |

2. Use a vacuum concentrator at a temperature of ≤60°C to concentrate to dryness;
3. After concentration, centrifuge at 12,000 rpm for 1 min. The prepared probes can be stored overnight at room temperature (15-25°C) for subsequent DNA library hybridization capture.

### Example 3: Use and Detection Method of the 10K Liquid-Phase Chip

This example demonstrates the operational process for using the 10K liquid-phase chip of the invention for genotyping. The specific steps are as follows:

### Step 1: Obtaining and extracting genomic DNA from the pig sample;

Ear tissue was collected from 133 pigs from a certain farm, including Large White and Landrace pigs, for genomic DNA extraction. The specific method is as follows:
1. Shred the appropriate amount of ethanol-dehydrated pig ear tissue and place it in a 96-well deep plate (use a 2.0mL centrifuge tube if the amount is small). Add a 5mm steel bead, freeze with liquid nitrogen, and grind with a grinder for 1-2 minutes.
2. Add 500 µL Buffer PL2 and 5 µL Proteinase K (the diluted Proteinase K currently used in the lab) to the deep-well plate. Secure the cap and mix well using a shaker.
3. Incubate at 65°C for 30 min, periodically invert the plate for mixing during incubation.
4. Add 500 µL phenol-chloroform-isoamyl alcohol to the deep-well plate, mix well by shaking or pipetting up and down, and let it stand for 5 min.
5. Centrifuge at 4000 rpm for 10 min, transfer 400 µL of the supernatant to a new 96-well deep-well plate. (Ensure not to aspirate the middle sediment).
6. Add 800 µL PW solution and mix well.
7. Transfer the supernatant from step 6 to a 96-well centrifuge column in two batches, and perform vacuum filtration.
8. Add 600 µL WB I to the 96-well centrifuge column, incubate at room temperature for 2 min, and perform vacuum filtration. (Ensure anhydrous ethanol has been added to WB I as specified on the bottle).
9. Add 600 µL WB II to the 96-well centrifuge column and perform vacuum filtration. (Ensure anhydrous ethanol has been added to WB II as specified on the bottle).
10. Add 600 µL WB II to the 96-well centrifuge column and perform vacuum filtration.
11. Place the 96-well centrifuge column into an empty collection plate, centrifuge at 4000 rpm for 5 min. Place the 96-well centrifuge column on a new 96-well PCR plate and air dry at room temperature.
12. Add 60-100 µL preheated 65°C TE to the 96-well centrifuge column, incubate at room temperature for 2 min, and centrifuge at 4000 rpm for 5 min (preheating the TE to 65°C helps improve DNA elution efficiency and gel electrophoresis detection of target fragment length).

### Step 2: Constructing a pig cDNA library;

### 1. Probe Mixture

a) In a PCR tube, prepare the following reaction using the invention's reagents: DNA 1 ng-200 ng, GenoBaits end repair buffer 4 µL, GenoBaits end repair enzyme 3.1 µL, add nuclease-free water to 20 µL.
b) After gently mixing the reaction system, briefly centrifuge to collect the reaction liquid at the bottom of the tube.
c) Place the reaction tube in a PCR machine and perform the following reaction, 37°C for 20 min, 72°C for 20 min, then hold at 4°C. Afterward, set the hot lid temperature to 82°C:

### 2. Adaptor ligation

a) Add the following components directly to the reaction system from step 1: GenoBaits ULtraDNA ligase 2 µL, GenoBaits ULtraDNA ligase buffer 8 µL, GenoBaits Adapter for MGI 2 µL, add nuclease-free water to a final volume of 20 µL.
b) After gently mixing the reaction system, briefly centrifuge to collect the reaction liquid at the bottom of the tube.
   Note: The system must be thoroughly mixed; otherwise, library construction may fail.
c) Place the reaction tube in a PCR machine and perform the following reaction program: 22°C for 60 min, hold at 4°C, and then turn off the hot lid:

### 3. DNA Purification

a) Take out GenoPrep DNA Clean Beads in advance and equilibrate at room temperature for over 30 min; vortex to mix before use.
b) Add 48 µL GenoPrep DNA Clean Beads to the ligation system from step 2, mix by vortexing, avoiding bubbles as much as possible; let stand for 5 min, and then briefly centrifuge to collect the liquid at the bottom of the tube.
c) Place the tube on a magnetic rack for at least 3 min until the solution is clear; remove the supernatant.
d) Keep the PCR tube on the magnetic rack, add 100 µL of 80% ethanol. Incubate at room temperature for 30 seconds, remove the supernatant.
e) Keep the PCR tube on the magnetic rack, open the cap and air dry for 5 minutes until the ethanol evaporates completely.
f) Remove the PCR tube from the magnetic rack and resuspend the beads with the PCR system in Step 4.

### 4. Library Amplification

When the starting amount is 1 ng-10 ng, 8 amplification cycles are recommended; for a starting amount of 10 ng-100 ng, 6-8 amplification cycles are recommended; for a starting amount of 100 ng and above, 6 cycles are recommended. :
a) In a new tube, prepare the following reaction using the reagents in this invention: GenoBaits PCR Master Mix 10 µL, I5 Barcode (10 µm) -MGI 1 µL, I7 Barcode (2 µm) -MGI 5 µL, add nuclease-free water to a final volume of 20 µL.
b) Add the above system to the beads dried in step 3, resuspend the beads, and briefly centrifuge to collect the reaction liquid at the bottom of the tube.
c) Place the reaction tube in a PCR machine and perform the following reaction:

| | | |
|---|---|---|
| 98°C | 2 min | |
| 98°C | 30 s | 6-8 cycles. |
| 50°C | 30 s | |
| 72°C | 40 s | |
| 72°C | 4 min | |

### 5. Purification

a) Add 20 µL GenoPrep DNA Clean Beads to the system from step 4, mix by vortexing, avoiding bubbles as much as possible; let stand for 5 min, and then briefly centrifuge to collect the liquid at the bottom of the tube.
b) Place the tube on a magnetic rack for at least 3 min until the solution is clear; remove the supernatant.
c) Keep the PCR tube on the magnetic rack, add 100 µL of 80% ethanol. Incubate at room temperature for 30 seconds, remove the supernatant.
d) Keep the PCR tube on the magnetic rack, and air-dry with the cap open for 10 minutes.
e) Remove the PCR tube from the magnetic rack, add 35 µL Tris-HCl, vortex to mix, let stand for 5 min, and then briefly centrifuge to collect the liquid at the bottom of the tube.
f) On the magnetic rack, wait until the solution clears (about 3 minutes), and transfer the supernatant to a new tube, store at -20 °C.
g) The library requires further quality testing (e.g., concentration measurement and distribution assessment) for subsequent sequencing or the next step of the experiment.

### Step 3: Hybridization of pig genomic fragments with the probe of this invention, PCR of the samples, and purification, followed by sequencing;

1. Use the mixed probe of this invention, melt at room temperature (15-25°C), mix well, and briefly centrifuge
2. GenoBaitsBlock II, GenoBaitsBlock
a) Depending on the library type, mix the following reagents in a 1.5 mL PCR tube: 0.6-1 µg of mixed library, 5 µg (5 µL) of GenoBaits Block I, 2 µL of GenoBaits Block II for ILM/MGI, and 300 ng of the probes of the present invention.
b) Use a vacuum concentrator at a temperature of ≤60°C to concentrate to dryness;
c) After concentration is complete, centrifuge at 12000 rpm for 1 min, and then proceed with subsequent operations.

3. Hybridization capture of the DNA library
a) Dissolve all GenoBaits hybridization reagents at room temperature;
b) Add the reagents to the tube;
c) Pipette or vortex to mix well, centrifuge at 12000 rpm for 1 min, let stand at room temperature for 5 min, pipette or vortex to mix again, lightly centrifuge, and transfer the entire mix to a 0.2 mL EP tube;
d) Thermal cycling incubation conditions: 95°C for 10 min (lid temperature at 105°C);
e) Once the PCR cycler cools down to 65°C, transfer it to another PCR machine with a lid temperature of 75°C and 65°C for hybridization. Note: If necessary, the experiment can be conducted overnight at 65°C (14-16h). *Hybridization at 65°C helps improve capture efficiency.

4. Preparation of elution buffer (Wash Buffer)
Single capture system, dilute GenoBaits buffers to 1× system.
*The 1× system can be stored at room temperature for up to 4 weeks.

| | Buffer | H₂O | Total volume |
|---|---|---|---|
| GenoBaits 2× Beads Wash Buffer V3.3 | 250 µL | 250 µL | 500 µL |
| GenoBaits 10 × Wash Buffer I V3.3 | 28 µL | 252 µL | 280 µL |
| GenoBaits 10 × Wash Buffer II V3.3 | 18 µL | 162 µL | 180 µL |
| GenoBaits 10 × Wash Buffer III V3.3 | 18 µL | 162 µL | 180 µL |
| GenoBaits 10 × Stringent Wash Buffer V3.3 | 40 µL | 360 µL | 400 µL |

*Precipitation may occur in 10 × Wash Buffer I and 10 × S-W Buffer at room temperature and can be dissolved by heating at 65°C.
5. Preparation of GenoBaits DNA Probe Beads
a) Place GenoBaits Probe Beads at room temperature for 10 min before use;
b) Vortex for 15 s to mix evenly;
c) Prepare 50 µL of GenoBaits Probe Beads for each reaction, place them in a 0.2 mL EP tube;
d) Place the tube on a magnetic rack, allowing the beads to fully separate from the solution.
e) Remove the supernatant, and retain the magnetic beads
f) Elution: For each reaction, add 150 µL of GenoBaits 1X Bead Wash Buffer, vortex for 10 seconds, transfer the tube to the magnetic rack, let the beads fully separate from the solution, and remove the supernatant.
g) Repeat step 6 above twice for a total of three washes.

6. Binding of hybridized fragments with GenoBaits DNA Probe Beads
a) Transfer the entire 16 µL of hybridization solution to the prepared beads
b) Vortex for 10 seconds to mix well, centrifuge for 5 seconds.
c) Place the EP tube in the PCR machine at 65°C for 45 minutes, with a heat cover temperature of 75 °C (to bind DNA with the beads)
a) Shake for 5 s every 12 min.

7. Elution to remove unbound DNA (using 1× Wash Buffer from step 4)
a) Prepare a 65°C elution buffer (completed on the PCR machine)
b) Prepare room temperature elution buffer
c) Resuspend the beads, the suspension is used for step 8, and keep the remaining 10µL as a backup.

8. PCR enrichment
a) According to the library type, prepare PCR reagents in a 0.2 mL PCR tube
b) Briefly vortex, centrifuge, and ensure the beads are still in the solution
c) Place the PCR tube in the PCR machine, with the heat cover temperature at 105°C, for PCR amplification

9. PCR Product Purification
a) Add 45 µL (1.5X volume) GenoPrep DNA Clean Beads to each PCR reaction, mix by vortexing, avoiding bubbles as much as possible; let stand for 5 min, and then briefly centrifuge to collect the liquid at the bottom of the tube.
b) Place the tube on a magnetic rack for at least 3 min until the solution is clear; remove the supernatant.
c) Keep the PCR tube on the magnetic rack, add 100 µL of 80% ethanol. Incubate at room temperature for 30 seconds, remove the supernatant.
d) Keep the PCR tube on the magnetic rack, and air-dry with the cap open for 10 minutes.
e) Remove the PCR tube from the magnetic rack, add 35 µL Tris-HCl, vortex to mix, let stand for 5 min, and then briefly centrifuge to collect the liquid at the bottom of the tube.
f) On the magnetic rack, wait until the solution clears (about 3 minutes), and transfer the supernatant to a new tube, store at -20 °C.
g) The library requires further quality testing (e.g., concentration measurement and distribution assessment) for subsequent sequencing or the next step of the experiment.

10. Library Testing
a) Measure the library with Qubit Fluorometer and Qubit dsDNA HS Assay Kit
b) Measure the average length of captured DNA library fragments on a digital electrophoresis system
c) Measure the library concentration with a KAPA Library Quantification Kit

11. Sequencing
Operate according to the requirements of the sequencing instrument. The average sequencing depth for target site SNPs is 105.88X.

### Step 4: mSNP Genotyping

Genotypes for all mSNP sites are obtained according to the sequencing data processing workflow. The specific steps are as follows:
1. Use Trimmomatic software to remove adapters and low-quality reads.
2. Use BWA software to align the reads of each individual to the pig reference genome Sscrofa11.1 (GCA_000003025.6);
3. Use SAMtools to generate BAM and sorted BAM files;
4. Use the GATK pipeline to generate a VCF file containing all mSNPs (including target site SNPs).

Example 3 demonstrates the operational process for using the liquid-phase chip of this invention, while Example 4 evaluates the genotyping quality of the liquid-phase chip in samples from pig farms.

### Example 4: Evaluation of Genotyping Quality of 10K mSNP Liquid-Phase Chip

Blood samples were collected from 133 Large White and Landrace pigs from a pig farm, and genotyping was performed using the invention according to the procedure described in Example 3 to evaluate the quality of mSNP markers of the invention.

### 1. Statistics on the Number of Target Sites and mSNP Markers

According to the genotype testing results of 133 pig samples on the 10K chip, the actual measurements are as follows: The 10K mSNP liquid phase chip has a total of 10,200 target SNP loci. After genotyping, there are 20,585 SNPs in total, which include 10,200 target SNPs and 10,385 mSNP markers within the probes for these target loci. This indicates that the mSNP liquid phase chip designed by the present invention can significantly increase the number of SNPs. Compared to solid-phase chips that only consider target loci, it can greatly increase the number of effective SNP markers without increasing sequencing costs.

### 2. Statistical Analysis of Chromosome Density Distribution of Target Sites and mSNP Markers

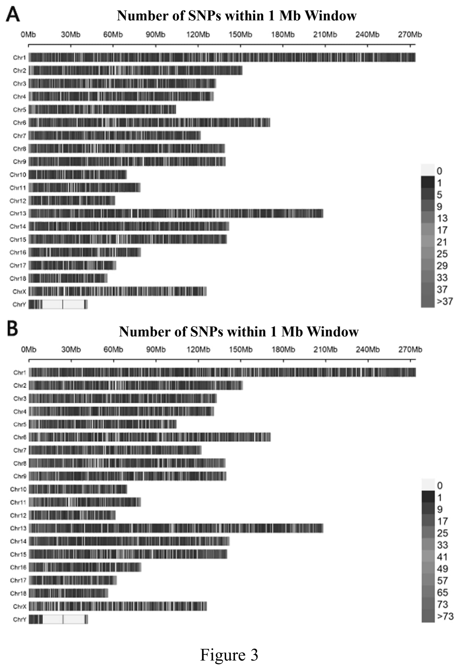

Figures 3A and 3B reflect the chromosome density distribution of target sites and mSNPs (including target site SNPs) on the 10K liquid-phase chip, showing a similar distribution pattern. mSNPs only increase SNP density on the basis of target site SNPs without altering the overall distribution pattern of SNPs. This indicates that in practical applications, the mSNP detection conforms to the features of theoretical test of multiple single nucleotide-polymorphism, thereby improving the efficiency of capturing effective mSNP markers.

### 3. Statistical Analysis of Missing Rates and MAF of Target Sites and mSNP Markers

The genotyping data of 133 samples show that there is almost no difference between the detection rate and minor allele frequency MAF of 10200 target sites and 10385 additional mSNPs in the mSNP liquid-phase chip (Table 2), indicating that the increase in SNP numbers due to the mSNP liquid-phase chip does not reduce chip data quality.

**Table 2 Genotype Quality of Target Site SNPs and mSNPs**

| Marker Type | Number of Markers | Proportion of Minor Allele Frequency < 0.05 | Detection Rate |
|---|---|---|---|
| Target Site SNPs | 10200 | 10.56% | 99.78% (0.029) |
| mSNPs | 10385 | 10.56% | 99.74% (0.031) |
| All SNPs | 20585 | 10.56% | 99.75% (0.030) |

| | | | |
|---|---|---|---|
| Note: Standard deviation is shown in parentheses | | | |

### 4. After Genotype Quality Control, Statistical Analysis of Target Sites and mSNP Markers;

Genotype quality control is a routine operation after high-throughput genotyping, primarily used to eliminate markers and individuals with low genotyping quality for subsequent genetic analysis or molecular breeding. The quality control steps are usually as follows:
a) Remove sites with unknown positions
b) Remove SNPs with a call rate below 90% or a missing rate higher than 10%;
c) Remove SNPs with a minor allele frequency (MAF) below 0.05;
d) Remove SNPs that significantly deviate from Hardy-Weinberg equilibrium (P < 10⁻⁶).

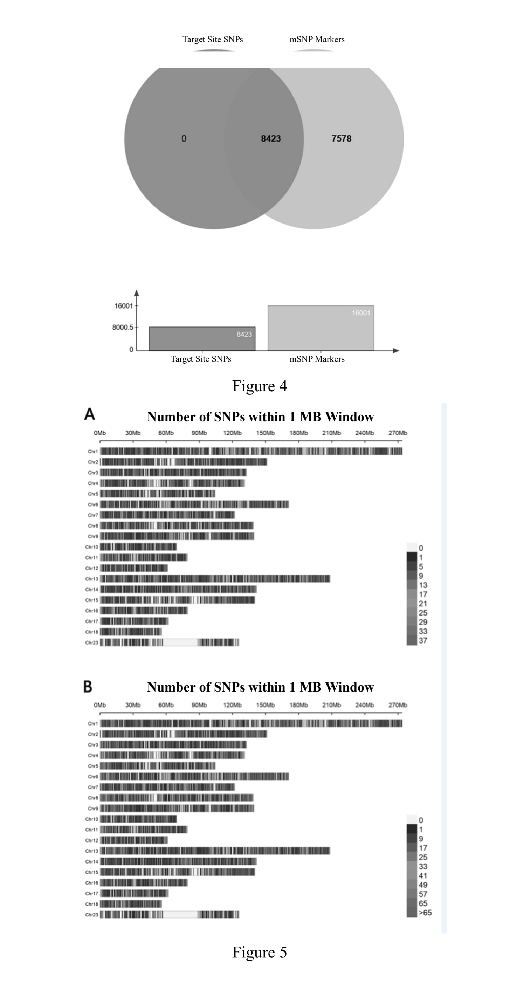

After quality control, some SNPs were removed from both the target site SNPs and all mSNP markers (including the target site) of the 10K mSNP liquid-phase chip. After quality control, there were a total of 16,001 mSNP markers, including 8,423 target loci (Figure 4). Target loci that did not meet quality control standards had mSNPs within their probes that also failed to meet quality control conditions. After quality control, the number of mSNP markers is still approximately twice that of the target loci, remaining essentially the same as before quality control. There was no significant change in the chromosome density distribution of target sites and all mSNP markers after quality control (Figures 5A, B), consistent with the distribution before quality control (Figures 3A, B). This indicates that mSNPs can significantly increase the number of SNPs detected without reducing data quality after quality control, which helps retain more genetic variation.

The main aim of the present invention is to reduce the cost of pig molecular breeding. Fewer markers mean lower costs. The detection cost of the present invention is one-third of that of the 50K solid-phase chip and half of that of the 50K liquid phase chip (ZL202110359470.7). However, genome selection is still more effective with 50,000 markers. The 10K liquid-phase chip primarily relies on genotype imputation technology to fill it up to the genotyping data of a 50K chip. Example 5 is provided to illustrate the accuracy of filling the 10K chip genotyping data up to a 50K chip and the effectiveness of genomic selection.

### Example 5: Accuracy of Genotype Imputation from 10K mSNP Liquid-Phase Chip to 50K Chip and Genomic Selection Effect

In this invention, the 10K liquid-phase chip genotyping was performed on the above 133 individuals, and genotyping was also performed using the applicant's 50K liquid-phase chip (ZL202110359470.7), ensuring that each individual had both 10K liquid-phase and 50K liquid-phase genotyping data.

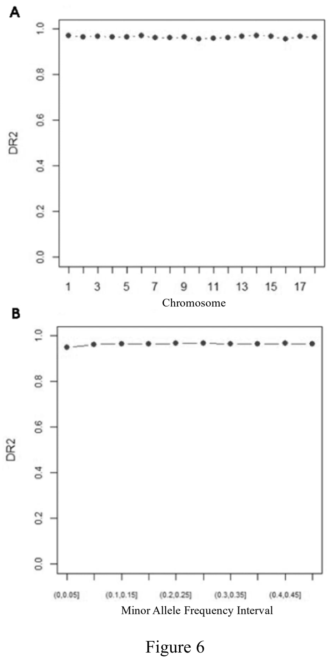

Theoretical research and breeding practice have shown that the 50K chip balances the effect of genomic selection and breeding costs and is the most widely used. To ensure the effectiveness of genomic selection with the 10K chip, genotype imputation technology is needed to fill the 10K chip data up to 50K chip genotyping data, so a sufficiently large 50K chip population must be constructed as a reference population for imputation, based on which the 10K chip can be filled. In this example, a pre-existing population of 4126 individuals was used as the reference population, including three breeds: Landrace, Large White, and Duroc, and they were genotyped using the 50K liquid-phase chip. It should be noted that the reference population for genotype imputation does not need to be very large; typically, 4000 individuals can ensure high imputation accuracy. Using the classic genotype imputation software Beagle 4.1, follow the operating guide to impute the 133 individual 10K liquid phase chip genotypes into the 50K liquid phase chip. Since these 133 individuals were also tested using the 50K liquid phase chip (original 50K chip genotypes), calculate the correlation coefficient between the imputed 50K chip genotypes for each site and the original 50K chip genotypes for these 133 samples. This correlation coefficient is used as a measure of imputation accuracy. As shown in Figure 6, the average imputation accuracy across all chromosomes was 0.964 (Figure 6). When the marker MAF was below 0.05, imputation accuracy was lower, but generally, such markers are removed during genotype quality control. Therefore, after genotype quality control, the accuracy of imputing 10K liquid phase chip genotypes into the 50K liquid phase chip will further increase to 0.98. Since the proportion of incorrectly imputed markers is low, it does not affect the genomic selection accuracy of the 50K liquid phase chip.

Based on the imputation from the 10K chip to the 50K chip in 133 individuals, this example further selected a larger population of 10K liquid-phase chips for imputation to the 50K liquid-phase chip to evaluate the effectiveness of genomic selection. In the example, 2,991 Large White pigs from the same farm were selected, including 1,199 individuals with phenotypic records for growth traits (age at 100 kg body weight and live backfat thickness at 100 kg body weight), 804 individuals with reproductive trait (total number of piglets born) records, and a total of 42,281 pedigree records. All individuals were genotyped using both the 50K liquid-phase chip and the 10K liquid-phase chip developed by this invention. The 10K liquid-phase chip was imputed to the 50K chip genotype data using the above-mentioned 4126 50K liquid-phase chip reference population. For validation, 198 individuals from the growth phenotype group and 108 individuals from the reproduction phenotype group were selected as the youngest individuals, with the remaining population serving as the reference group. The genomic estimated breeding values for the three traits were estimated using the classical single-step GBLUP (ssGBLUP) method. The accuracy of genomic selection was evaluated by the Pearson correlation coefficient between the phenotypic values corrected for traits in the validation group and their estimated genomic breeding values. The results indicate that, as shown in Table 3, there is no difference in genomic selection accuracy between the imputed 50K chip and the original 50K chip for the three traits. This demonstrates that using only the 10K liquid phase chip for detection and imputing it to the 50K liquid phase chip yields the same genomic selection effectiveness as using the 50K liquid phase chip alone.

**Table 3. Accuracy of Genotype Imputation from 10K Liquid-Phase Chip to 50K Liquid-Phase Chip for Genomic Selection**

| Trait | Reference Population Size (Head) | Validation Population (Head) | Accuracy | |
|---|---|---|---|---|
| | | | 50K Liquid-Phase | Genotype Imputation from 10K to 50K |
| Age at 100 kg Body Weight | 1001 | 198 | 0.352 | 0.350 |
| 100 kg Body Weight Backfat Thickness | 1001 | 198 | 0.307 | 0.302 |
| Total Number of Piglets Born | 696 | 108 | 0.325 | 0.323 |

In genomic selection implementation, a large number of individual genotyping tests are required. Using the 50K liquid phase chip would result in high genotyping costs. However, with genotype imputation, only a certain number of individuals need to be genotyped with the 50K liquid phase chip to serve as a reference group for imputation, allowing for more extensive use of the 10K chip developed by the present invention. The present invention achieves the goal of significantly reducing the cost of genomic selection in molecular breeding without compromising genomic selection accuracy by using genotype imputation technology with the 10K liquid phase chip. This solves a bottleneck in molecular breeding in China's pig industry, enabling large-scale implementation of molecular breeding, and becoming a booster for the revitalization of China's seed industry.

## Claims

1. A method for SNP marker selection and probe preparation for a 10K liquid phase chip used for multiple single nucleotide-polymorphism, which utilizes whole-genome sequencing data of pig breeds to mine and screen target SNP loci, then designs and optimizes probes for the target SNP loci, ultimately resulting in the determination of the probes. The method includes the following steps:
Step 1: Determining target SNPs:
Target SNPs are selected as determined target SNPs using the following method :(1) Preliminary screening of target SNP markers: Based on whole-genome sequencing data from Duroc, Large White, and Landrace pig breeds, aligned to the pig 11.1 reference genome. Markers are selected for targeted capture sites, i.e., target SNPs, based on marker spacing and gene function annotations. The principles for selecting target SNPs are: ① Uniform distribution across chromosomes, with denser distribution at both ends of chromosomes; ② Marker spacing greater than 280Kb; ③ Important candidate loci for growth, reproduction, feed efficiency, meat quality, and body size traits; ④ SNP markers preferably located in QTL regions related to economic traits, as determined by comparison with the QTLdb database. Markers obtained in this manner are used as target SNPs.
(2) Supplementing and optimizing target SNPs: Based on the target SNPs obtained in the preliminary screening step, supplement and optimize markers with high polymorphism and moderate regional linkage disequilibrium. The principles are as follows: ① Polymorphism primarily considers MAF > 0.35 in Duroc, Landrace, and Large White pigs; ② The average linkage disequilibrium (r²) with adjacent SNP markers is below 0.85.
Step 2, designing probes based on the determined target site SNPs: Utilize the multiple single nucleotide polymorphism technologies to design 1-4 probes, each 110 bp in length, centered on each target SNP. Each probe covers the target SNP, with a total probe coverage of 165 bp around the target SNP. The principles for probe design are: 1) Select probes with a content between 30% and 80%; 2) Choose regions with a number of homologous areas ≤ 5; 3) Select probe areas that do not contain SSR, N regions.
Step 3, selecting and optimizing probes containing high-quality mSNPs: Probes are hybridized and sequenced, and the genotyping quality of mSNPs, including target sites, is detected. Set a missing rate of NA<0.1, a minimum allele frequency (MAF) ≥0.05, and heterozygosity (Het) <0.5 as standards to screen mSNPs, removing those that do not meet the standards. If the probe does not meet the genotype quality control requirements of mSNPs, delete the probe and the corresponding target site SNP. The mSNPs that meet the quality inspection requirements are finally used as the target sites of the 10K mSNP liquid-phase chip.

2. The method according to claim 1, wherein the whole-genome sequencing data of pig breeds is based on the 11.1 reference genome; the principles for selecting target SNPs are: ① Uniform distribution across chromosomes, with denser distribution at both ends of the chromosomes; ② Polymorphism considers MAF > 0.35 in Duroc, Landrace, and Large White pigs; ③ Average linkage disequilibrium (r2) with upstream and downstream SNP markers less than 0.85; ④ Comparison with the QTLdb database, aiming for SNP markers to be located in QTL regions related to economic traits; ⑤ Overlap with some loci on the known 50K chip for pigs.

3. The method according to claim 2, wherein the known pig 50K chip is a 50K SNP liquid phase chip, GGP50K from Neogen Corporation, or Zhongxin No. 1.

4. A 10K mSNP liquid-phase chip for pigs based on multiple single nucleotide polymorphisms, **characterized in that** it is prepared from probes obtained by the method according to any of claims 1 to 3.

5. The pig 10K mSNP liquid phase chip according to claim 4, wherein after synthesizing the probes, they are mixed in equal molar amounts, diluted to 1-5 pmol/mL in the buffer solution, and then prepared into the probe hybridization solution.

6. The 10K mSNP liquid-phase chip for pigs according to claim 5, **characterized in that** the buffer solution is a mixture of EDTA and Tris-HCl.

7. The 10K mSNP liquid-phase chip for pigs according to claim 5, **characterized in that** it further includes using a Pooled, barcoded library, GenoBaits Block I, and GenoBaits Block II for ILM/MGI to prepare the probe hybridization solution with the following components:
| Component name | Quantity |
|---|---|
| Pooled, barcoded library | 0.6 µL |
| GenoBaits Block I | 5 µL |
| GenoBaits Block II | 2 µL |
| forILM/MGI | |
| The said probe | 300 ng. |

8. The 10K mSNP liquid-phase chip for pigs according to claim 7, **characterized in that** the probe hybridization solution is concentrated to dryness using a vacuum concentrator at a temperature ≤60°C.

9. A method for detecting the genotype of individual pigs using the 10K mSNP liquid-phase chip for pigs according to any of claims 4 to 8. The said method includes the following steps: obtaining samples from the pigs to be tested and extracting genomic DNA; constructing pig cDNA libraries; hybridizing and sequencing the constructed libraries with the pig 10K mSNP liquid-phase chip; performing mSNP genotyping according to the sequencing data operation process, and determining the genotypes of all liquid-phase chip marker loci for each individual.
